# EUROPEAN PATENT APPLICATION

(11) **EP 4 302 740 A1**
(43) Date of publication of application: **10.01.2024**
(21) Application number: 22216823.9
(22) Date of filing: 27.12.2022
(51) Int. Cl.: A61F 13/511, A61F 13/514, A61F 13/53

(54) **ABSORBENT ARTICLE FOR PET EXCRETA**

(30) Priority: 05.07.2022 TW 111125173
(71) Applicant: KNH Enterprise Co., Ltd., Tainan City 72256 (TW)
(72) Inventor: SU, Chien-Chung, 72256 Tainan City (TW); TSENG, Yu-Hsuan, 72256 Tainan City (TW); LIU, Yun-Peng, 72256 Tainan City (TW)
(74) Representative: V.O.

(57) **Abstract**

An absorbent article for pet excreta includes a liquid-permeable topsheet layer (2), a liquid-permeable backsheet layer (3), and a liquid-absorbing unit (4). The liquid-permeable backsheet layer (3) is disposed spaced apart from the liquid-permeable topsheet layer (2). The liquid-absorbing unit (4) is disposed between the liquid-permeable topsheet layer (2) and the liquid-permeable backsheet layer (3), and is adapted for absorbing pet excreta from at least one of the liquid-permeable topsheet layer (2) and the liquid-permeable backsheet layer (3).

## Description

The present disclosure relates to an article for pets, and more particularly to an absorbent article for pet excreta.

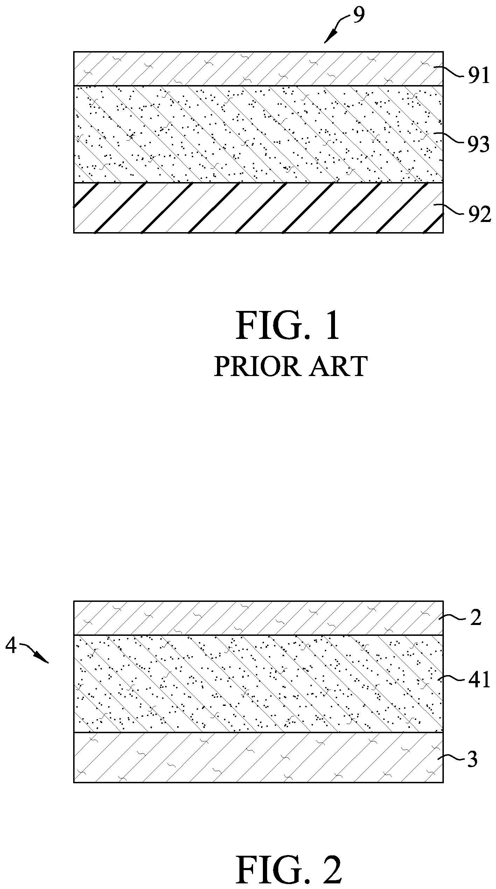

Referring to FIG. 1, in order to prevent pet excreta from soiling the floor, a conventional absorbent article for pets generally includes a liquid-permeable topsheet layer 91, a liquid-impermeable backsheet layer 92 that is disposed spaced apart from the liquid-permeable topsheet layer 91, and a liquid-absorbing unit 93 that is disposed between the liquid-permeable topsheet layer 91 and the liquid-impermeable backsheet layer 92. Use of the conventional absorbent article is on the premise that pets would obediently urinate and/or defecate in a central area of the conventional absorbent article so as to allow pet excreta to pass through the liquid-permeable topsheet layer 91 and then enter into the liquid-absorbing unit 93 to be absorbed, and to prevent excessive pet excreta which is unable to be absorbed by the liquid-absorbing unit 93 from seeping out of the conventional absorbent article through the liquid-impermeable backsheet layer 92 and soiling the floor.

However, pets often would not stay in the central area of the conventional absorbent article during urination and/or defecation, and instead, might urinate and/or defecate at peripheral region of the conventional absorbent article, resulting in only a portion of the pet excreta to drop on the liquid-permeable topsheet layer 91, whereas another portion of the pet excreta dropped on the peripheral region of the conventional absorbent article would leak to the floor along outer periphery of the liquid-impermeable backsheet layer 92. Since the liquid-impermeable backsheet layer 92 prevents the leaked pet excreta from being absorbed by the liquid-absorbing unit 93, the floor would still be soiled with the pet excreta, causing pet owners a trouble of having to clean up the pet excreta on the floor using a cloth or a large amount of toilet papers.

Therefore, an object of the present disclosure is to provide an absorbent article for pet excreta that can alleviate at least one of the drawbacks of the prior art.

According to the present disclosure, the absorbent article for pet excreta includes a liquid-permeable topsheet layer, a liquid-permeable backsheet layer, and a liquid-absorbing unit. The liquid-permeable backsheet layer is disposed spaced apart from the liquid-permeable topsheet layer. The liquid-absorbing unit is disposed between the liquid-permeable topsheet layer and the liquid-permeable backsheet layer, and is adapted for absorbing pet excreta from at least one of the liquid-permeable topsheet layer and the liquid-permeable backsheet layer.

Other features and advantages of the present disclosure will become apparent in the following detailed description of the embodiment(s) with reference to the accompanying drawings. It is noted that various features may not be drawn to scale.
FIG. 1 is a sectional schematic view illustrating a conventional absorbent article for pets.
FIG. 2 is a sectional schematic view illustrating a first embodiment of an absorbent article for pet excreta according to the present disclosure.
FIG. 3 is sectional schematic view illustrating a variation of the first embodiment.
FIG. 4 is a sectional schematic view illustrating another variation of the first embodiment.
FIG. 5 is a sectional schematic view illustrating a second embodiment of the absorbent article for pet excreta according to the present disclosure.
FIG. 6 is a sectional schematic view illustrating a third embodiment of the absorbent article for pet excreta according to the present disclosure.
FIG. 7 is a sectional schematic view illustrating a fourth embodiment of the absorbent article for pet excreta according to the present disclosure.
FIG. 8 is a sectional schematic view illustrating a fifth embodiment of the absorbent article for pet excreta according to the present disclosure.
FIG. 9 is a sectional schematic view illustrating a sixth embodiment of the absorbent article for pet excreta according to the present disclosure.
FIG. 10 are photographs respectively showing the liquid absorption results of the absorbent articles for pet excreta of Examples 1 to 4 and Comparative Example 1.

Before the present disclosure is described in greater detail, it should be noted that where considered appropriate, reference numerals or terminal portions of reference numerals have been repeated among the figures to indicate corresponding or analogous elements, which may optionally have similar characteristics.

It should be noted herein that for clarity of description, spatially relative terms such as "top," "bottom," "upper," "lower," "on," "above," "over," "downwardly," "upwardly" and the like may be used throughout the disclosure while making reference to the features as illustrated in the drawings. The features may be oriented differently (e.g., rotated 90 degrees or at other orientations) and the spatially relative terms used herein may be interpreted accordingly.

Referring to FIG. 2, a first embodiment of an absorbent article for pet excreta according to the present disclosure includes a liquid-permeable topsheet layer 2, a liquid-permeable backsheet layer 3, and a liquid-absorbing unit 4. The liquid-permeable backsheet layer 3 is disposed spaced apart from the liquid-permeable topsheet layer 2. The liquid-absorbing unit 4 is disposed between the liquid-permeable topsheet layer 2 and the liquid-permeable backsheet layer 3, and is adapted for absorbing pet excreta from at least one of the liquid-permeable topsheet layer 2 and the liquid-permeable backsheet layer 3. The liquid-absorbing unit 4 includes a liquid-absorbing body 41 that is adapted for absorbing pet excreta.

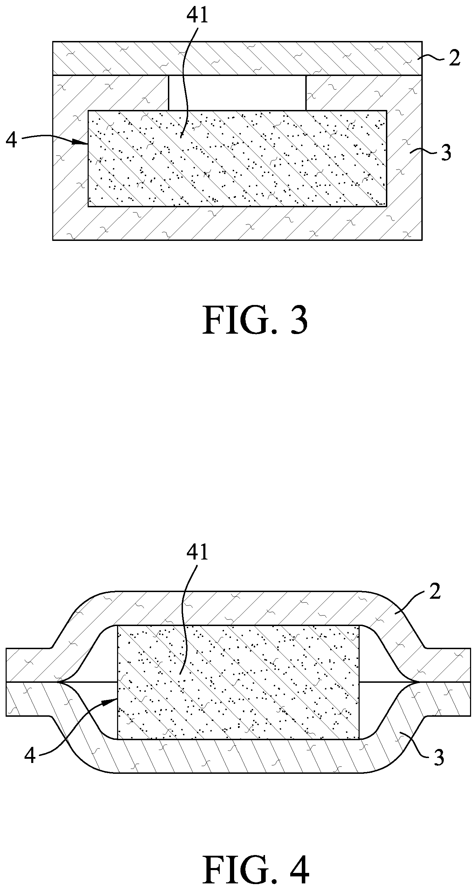

The liquid-permeable topsheet layer 2 and the liquid-permeable backsheet layer 3 may be connected to each other and may be configured to enclose the liquid-absorbing unit 4, but is not limited thereto. For example, as shown in FIG. 3, in a variation of the first embodiment, the liquid-permeable backsheet layer 3 is formed to define a pocket-like open space for accommodating the liquid-absorbing unit 4, and the liquid-permeable topsheet layer 2 seals the pocket-like open space and is connected to the liquid-permeable backsheet layer 3. Alternatively, as shown in FIG. 4, in another variation of the first embodiment, the liquid-permeable topsheet layer 2 and the liquid-permeable backsheet layer 3 extend beyond the liquid-absorbing unit 4, and are connected to each other at end portions thereof.

The absorbent article for pet excreta of the present disclosure is specifically designed based on pet behavior, and is suitable to be placed on, e.g., the floor for use when pets perform excretion, so as to absorb pet excreta (e.g., urine). Both of the liquid-permeable topsheet layer 2 and the liquid-permeable backsheet layer 3 allow the penetration of the pet excreta, so that the pet excreta, upon contacting with either the liquid-permeable topsheet layer 2 or the liquid-permeable backsheet layer 3, would enter into and be absorbed by the liquid-absorbing unit 4. Therefore, even if a pet stands on a peripheral region of the absorbent article of the present disclosure during excretion, causing not all of the pet excreta to fall on the liquid-permeable topsheet layer 2 and to enter into and be absorbed by the liquid-absorbing unit 4 (i.e., a portion of the pet excreta falls on the liquid-permeable topsheet layer 2 whereas another portion of the pet excreta falls outside of the liquid-permeable topsheet layer 2 and leaks to the floor along outer periphery of the liquid-permeable backsheet layer 3), the leaked portion of the pet excreta can still be absorbed by the liquid-absorbing unit 4 via the liquid-permeable backsheet layer 3. Since pet excreta can penetrate into both the liquid-permeable topsheet layer 2 and the liquid-permeable backsheet layer 3, the absorbent article of the present disclosure can be laid on the floor with either one of the liquid-permeable topsheet layer 2 or the liquid-permeable backsheet layer 3 facing up, thereby providing convenience in use.

The materials and properties of the liquid-permeable topsheet layer 2 and the liquid-permeable backsheet layer 3 are not particularly limited, as long as the liquid-permeable topsheet layer 2 and the liquid-permeable backsheet layer 3 allow liquids to penetrate therethrough. In certain embodiments, the liquid-permeable topsheet layer 2 is made of a fresh material and/or a recycled material (e.g., recycled fiber or recycled plastic, but not limited thereto), and includes at least one selected from the group consisting of hydrophilic woven fabric, hydrophilic spun-bond non-woven fabric, hydrophilic hot-press non-woven fabric, hot-air non-woven fabric, spun-lace non-woven fabric, and porous plastic film. In other embodiments, the liquid-permeable backsheet layer 3 is made of a fresh material and/or a recycled material (e.g., recycled fiber or recycled plastic, but not limited thereto), and includes at least one selected from the group consisting of hydrophilic woven fabric, hydrophilic spun-bond non-woven fabric, hydrophilic hot-press non-woven fabric, hot-air non-woven fabric, spun-lace non-woven fabric, and porous plastic film. The liquid-permeable topsheet layer 2 and the liquid-permeable backsheet layer 3 may be made of the same material or different materials. In a case that each of the liquid-permeable topsheet layer 2 and the liquid-permeable backsheet layer 3 is at least one selected from the group consisting of hydrophilic woven fabric, hydrophilic spun-bond non-woven fabric, hydrophilic hot-press non-woven fabric, hot-air non-woven fabric, spun-lace non-woven fabric, the liquid-permeable topsheet layer 2 and the liquid-permeable backsheet layer 3 are also capable of absorbing liquid, and thus, pet excreta can be further absorbed by the liquid-permeable topsheet layer 2 and the liquid-permeable backsheet layer 3, so as to enhance absorption capacity of the absorbent article of the present disclosure.

The material and properties of the liquid-absorbing unit 4 are not particularly limited, as long as the liquid-absorbing unit 4 is conferred with liquid-absorbing property. In certain embodiments, the liquid-absorbing body 41 of the liquid-absorbing unit 4 is made of a fresh material and/or a recycled material, and includes at least one selected from the group consisting of superabsorbent polymer sheet (abbreviated as SAP sheet), liquid-absorbing polymer powder, paper pulp, cotton, and polyurethane foam. The amount of liquid to be absorbed by the liquid-absorbing unit 4 is not particularly limited, and may be flexibly adjusted and designed according to the practical needs (e.g., the amount of pet excreta). For example, the liquid-absorbing unit 4 may be designed to absorb an amount of liquid that is much greater than the amount of pet excreta.

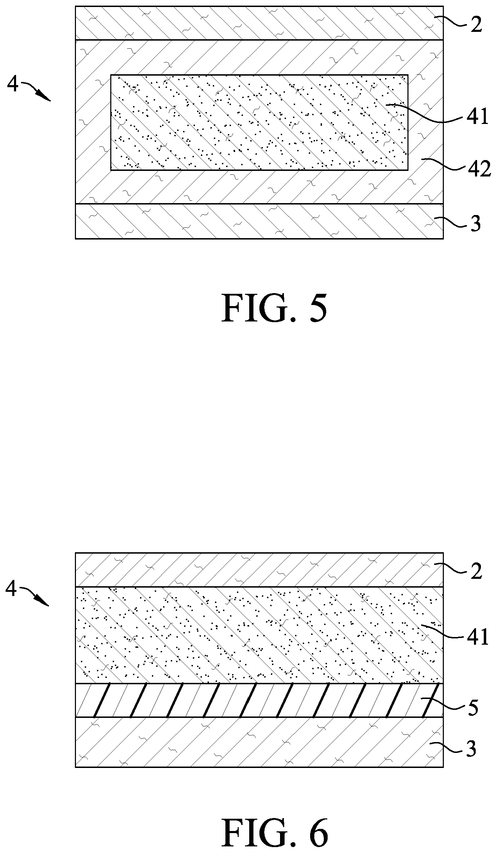

Referring to FIG. 5, a second embodiment of the absorbent article for pet excreta according to the present disclosure is generally similar to the first embodiment, except that in the second embodiment, the liquid-absorbing unit 4 further includes an absorbent paper 42 that encloses the liquid-absorbing body 41. Upon entering into the liquid-absorbing unit 4, the pet excreta is first absorbed by the absorbent paper 42, and the thus absorbed pet excreta would pass therethrough and then enter into the liquid-absorbing body 41 to be further absorbed, thereby enhancing the absorption capacity of the liquid-absorbing unit 4.

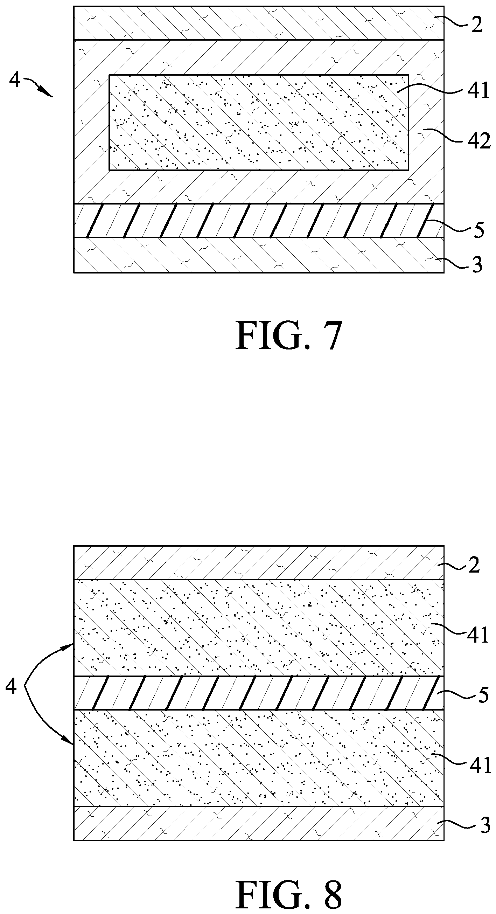

Referring to FIGS. 6 and 7, a third embodiment and a fourth embodiment of the absorbent article for pet excreta according to the present disclosure is generally similar to the first and second embodiments, respectively, except that in the third and fourth embodiments, the absorbent article further includes a liquid-impermeable leakproof layer 5 that is disposed between the liquid-absorbing unit 4 and the liquid-permeable backsheet layer 3. In the third and fourth embodiments, the liquid-permeable backsheet layer 3 is made of a fresh material and/or a recycled material (e.g., recycled fiber, but not limited thereto), and includes at least one selected from the group consisting of hydrophilic woven fabric, hydrophilic spun-bond non-woven fabric, hydrophilic hot-press non-woven fabric, hot-air non-woven fabric, and spun-lace non-woven fabric.

The material and properties of the liquid-impermeable leakproof layer 5 are not particularly limited, as long as the liquid-impermeable leakproof layer 5 is capable of preventing liquid from passing therethrough. For example, the liquid-impermeable leakproof layer 5 may include a waterproof plastic film. When an excessive amount of pet excreta falls on the liquid-permeable topsheet layer 2 (i.e., the absorption capacity of the liquid-absorbing unit 4 exceeds its loading capacity), the liquid-impermeable leakproof layer 5 is capable of preventing the pet excreta which leaks out from the liquid-absorbing unit 4 from passing therethrough, and the liquid-permeable backsheet layer 3, on the other hand, is capable of absorbing the pet excreta which accidently fall outside of the absorbent article.

Referring to FIG. 8, a fifth embodiment of the absorbent article for pet excreta according to the present disclosure is generally similar to the first embodiment, except that in the fifth embodiment, the liquid-absorbing unit 4 includes a plurality of the liquid-absorbing bodies 41 that are stacked on each other, and the absorbent article further includes the liquid-impermeable leakproof layer 5 that is disposed between two adjacent ones of the liquid-absorbing bodies 41. By including the liquid-absorbing bodies 41, absorption of pet excreta by the absorbent article of the present disclosure is further improved.

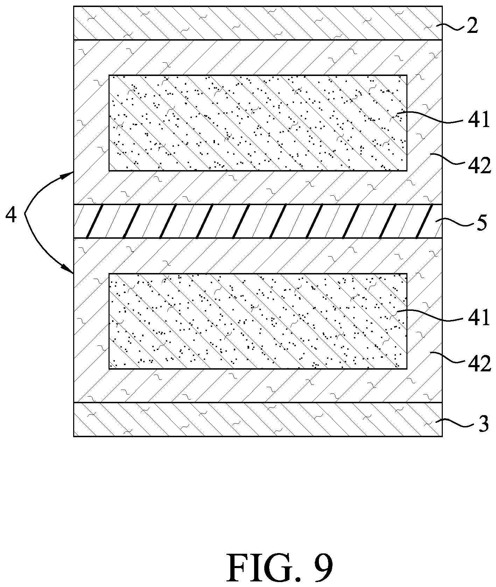

Referring to FIG. 9, a sixth embodiment of the absorbent article for pet excreta according to the present disclosure is generally similar to the fifth embodiment, except that in the sixth embodiment, the liquid-absorbing unit 4 further includes a plurality of the absorbent paper 42 each enclosing a respective one of the liquid-absorbing bodies 41.

The present disclosure will be described by way of the following examples. However, it should be understood that the following examples are intended solely for the purpose of illustration and should not be construed as limiting the present disclosure in practice.

### Example 1 (EX1)

The absorbent article for pet excreta of EX1 has a configuration as described for the variation of the aforesaid first embodiment shown in FIG. 4, and is made of the following materials. To be specific, in EX1, the liquid-permeable topsheet layer is made of a hydrophilic spun-bond non-woven fabric (Manufacturer: Foshan Nanhai Beautiful Nonwoven Co., Ltd., China) having a basis weight of 18 grams per square meter (gsm, g/m²), the liquid-absorbing unit is made of a superabsorbent polymer sheet (Manufacturer: KNH Enterprise Co., Ltd., Taiwan) having a basis weight of 190 gsm, and the liquid-permeable backsheet layer is made of a hydrophilic spun-bond non-woven fabric having a basis weight of 18 gsm.

### Example 2 (EX2)

The absorbent article for pet excreta of EX2 has a configuration identical to that of EX1 and is made of materials similar to those of EX1, except that in EX2, the liquid-permeable backsheet layer is made of a porous plastic film (Manufacturer: Xiamen Yanjan New Material Co., Ltd., China) having a basis weight of 22.5 gsm.

### Example 3 (EX3)

The absorbent article for pet excreta of EX3 has a configuration as described for the aforesaid third embodiment, and is made of the following materials. Specifically, in EX3, the liquid-permeable topsheet layer is made of a hydrophilic spun-bond non-woven fabric having a basis weight of 18 gsm, the liquid-absorbing unit that is made of a superabsorbent polymer sheet having a basis weight of 190 gsm, the liquid-impermeable leakproof layer is made of a waterproof plastic film having a basis weight of 22 gsm, and the liquid-permeable backsheet layer is made of a hot-air non-woven fabric having a basis weight of 35 gsm.

### Example 4 (EX4)

The absorbent article for pet excreta of EX4 has a configuration identical to that of EX3 and is made of materials similar to those of EX3, except that in EX4, the liquid-permeable backsheet layer is made of a spun-lace non-woven fabric having a basis weight of 30 gsm.

### Comparative Example 1 (CE1)

The absorbent article for pet excreta of CE1 has a configuration identical to that of the conventional absorbent article for pets as shown in FIG. 1, and is made of the following materials. To be specific, in CE1, the liquid-permeable topsheet layer is made of a hydrophilic spun-bond non-woven fabric having a basis weight of 18 gsm, the liquid-absorbing unit is made of a superabsorbent polymer sheet having a basis weight of 190 gsm, and the liquid-impermeable backsheet layer is made of a waterproof plastic film having a basis weight of 22 gsm.

The types of materials and basis weights thereof for making each component in a respective one of the absorbent articles of EX1 to EX4 and CE1 are shown in Table 1 below.

**Table 1**

| Absorbent article for pet excreta | | EX1 | EX2 | EX3 | EX4 | EX5 |
|---|---|---|---|---|---|---|
| Liquid-permeable topsheet layer | Material | Hydrophilic spun-bond non-woven fabric | Hydrophilic spun-bond non-woven fabric | Hydrophilic spun-bond non-woven fabric | Hydrophilic spun-bond non-woven fabric | Hydrophilic spun-bond non-woven fabric |
| | Basis weight (gsm) | 18 | 18 | 18 | 18 | 18 |
| Liquid-absorbing unit | Material | Superabsorbent polymer sheet | Superabsorbent polymer sheet | Superabsorbent polymer sheet | Superabsorbent polymer sheet | Superabsorbent polymer sheet |
| | Basis weight (qsm) | 190 | 190 | 190 | 190 | 190 |
| Liquid-impermeable leakproof layer | Material | None | None | Waterproof plastic film | Waterproof plastic film | None |
| | Basis weight (gsm) | | | 22 | 22 | |
| Liquid-permeable backsheet layer | Material | Hydrophilic spun-bond non-woven fabric | Porous plastic film | Hot-air non-woven fabric | Spun-lace non-woven fabric | None |
| | Basis weight (gsm) | 18 | 22.5 | 35 | 30 | |
| Liquid-impermeable backsheet layer | Material | None | None | None | None | Waterproof plastic film |
| | Basis weight (qsm) | | | | | 22 |

### Property evaluation

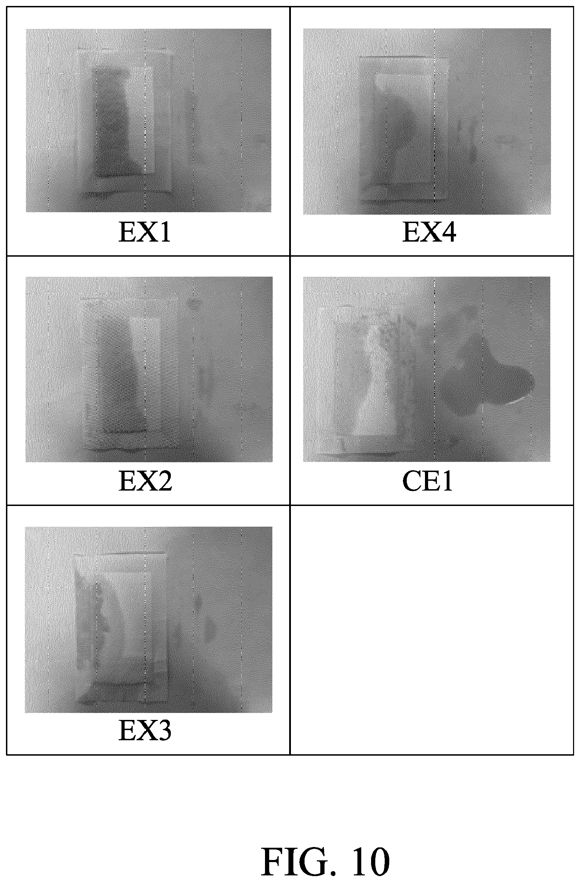

Each of the absorbent articles for pet excreta of EX1 to EX4 and CE1 was subjected to a liquid-absorption test to evaluate the liquid absorption capacity thereof. First, each of the absorbent articles of EX1 to EX4 and CE1 was placed on the floor with the liquid-permeable topsheet layer facing upwards, and then 5 pools of red-dyed water each having a volume of 20 mL were separately poured on a peripheral region of the absorbent article, such that a portion of the red-dyed water falls on the liquid-permeable topsheet layer of the absorbent article and another portion of the red-dyed water falls outside of the liquid-permeable topsheet layer and leaks out of the absorbent article. Next, each of the absorbent articles was left to stand for 1 minute, and then moved away and turned over such that the liquid-permeable topsheet layer was faced down. Then, the liquid absorption capacity of each absorbent article was determined by observing whether the red-dyed water remained on the floor. The results are shown in FIG. 10.

Referring to FIG. 10, as compared with the absorbent article of CE1, almost all of the leaked red-dyed water on the floor can be absorbed by the absorbent articles of EX1 to EX4 through the liquid-permeable backsheet layer (i.e., almost none of the red-dyed water remained on the floor), indicating that the absorbent articles of EX1 to EX4 are significantly better in absorbing the leaked liquid on the floor.

In particular, the liquid-absorbing units in the central region of the absorbent articles of EX1 and EX2 were dyed by absorbing the red-dyed water, indicating that the red-dyed water leaking outside the absorbent article can pass through the liquid-permeable backsheet layer and be effectively absorbed by the liquid-absorbing unit. As to the absorbent articles of EX3 and EX4, both the liquid-absorbing unit and the liquid-permeable backsheet layer were dyed by absorbing the red-dyed water, indicating that even in the presence of the liquid-impermeable leakproof layer, the liquid-permeable backsheet layer made of non-woven fabric having liquid-absorbing property is conducive to further improving the liquid absorption capacity of the absorbent articles of EX3 and EX4.

In summary, by disposing the liquid-permeable topsheet layer 2 and the liquid-permeable backsheet layer 3 on opposite surfaces of the liquid-absorbing unit 4, the absorbent article of the present disclosure allows pet excreta to enter into the liquid-absorbing unit 4 from the liquid-permeable topsheet layer 2 and/or from the liquid-permeable backsheet layer 3 so as to be absorbed by the liquid-absorbing unit 4. In addition, by having the liquid-permeable topsheet layer 2 and/or the liquid-permeable backsheet layer 3 made of a liquid-absorbing material, the pet excreta can also be absorbed by the liquid-permeable topsheet layer 2 and/or the liquid-permeable backsheet layer 3 so as to further improve the absorption capacity of the absorbent article of the present disclosure.

In the description above, for the purposes of explanation, numerous specific details have been set forth in order to provide a thorough understanding of the embodiment(s). It will be apparent, however, to one skilled in the art, that one or more other embodiments may be practiced without some of these specific details. It should also be appreciated that reference throughout this specification to "one embodiment," "an embodiment," an embodiment with an indication of an ordinal number and so forth means that a particular feature, structure, or characteristic may be included in the practice of the disclosure. It should be further appreciated that in the description, various features are sometimes grouped together in a single embodiment, figure, or description thereof for the purpose of streamlining the disclosure and aiding in the understanding of various inventive aspects; such does not mean that every one of these features needs to be practiced with the presence of all the other features. In other words, in any described embodiment, when implementation of one or more features or specific details does not affect implementation of another one or more features or specific details, said one or more features may be singled out and practiced alone without said another one or more features or specific details. It should be further noted that one or more features or specific details from one embodiment may be practiced together with one or more features or specific details from another embodiment, where appropriate, in the practice of the disclosure.

## Claims

1. An absorbent article for pet excreta, **characterized by**:
a liquid-permeable topsheet layer (2),
a liquid-permeable backsheet layer (3) that is disposed spaced apart from said liquid-permeable topsheet layer (2), and
a liquid-absorbing unit (4) that is disposed between said liquid-permeable topsheet layer (2) and said liquid-permeable backsheet layer (3) and that is adapted for absorbing pet excreta from at least one of said liquid-permeable topsheet layer (2) and said liquid-permeable backsheet layer (3).

2. The absorbent article as claimed in claim 1, **characterized in that** said liquid-permeable topsheet layer (2) is connected to said liquid-permeable backsheet layer (3), and said liquid-absorbing unit (4) is enclosed by said liquid-permeable topsheet layer (2) and said liquid-permeable backsheet layer (3).

3. The absorbent article as claimed in claim 1 or 2, **characterized in that** said liquid-absorbing unit (4) includes at least one liquid-absorbing body (41) that is adapted for absorbing pet excreta.

4. The absorbent article as claimed in any one of claims 1 to 3, further **characterized by** a liquid-impermeable leakproof layer (5) that is disposed between said liquid-absorbing unit (4) and said liquid-permeable backsheet layer (3).

5. The absorbent article as claimed in any one of claims 1 to 4, **characterized in that** said liquid-permeable backsheet layer (3) is made of one of a fresh material, a recycled material and a combination thereof, and includes at least one selected from the group consisting of hydrophilic woven fabric, hydrophilic spun-bond non-woven fabric, hydrophilic hot-press non-woven fabric, hot-air non-woven fabric, and spun-lace non-woven fabric.

6. The absorbent article as claimed in any one of claims 1 to 3, **characterized in that** said liquid-absorbing unit (4) includes a plurality of liquid-absorbing bodies (41) that are stacked on each other.

7. The absorbent article as claimed in claim 6, **characterized in that** said liquid-impermeable leakproof layer (5) is disposed between any two adjacent ones of said liquid-absorbing bodies (41).

8. The absorbent article as claimed in any one of claims 1 to 7, **characterized in that** said liquid-absorbing unit (4) further includes at least one absorbent paper (42) that encloses at least one of said liquid-absorbing bodies (41).

9. The absorbent article as claimed in any one of claims 1 to 8, **characterized in that** said liquid-permeable topsheet layer (2) is made of one of a fresh material, a recycled material and a combination thereof, and includes at least one selected from the group consisting of hydrophilic woven fabric, hydrophilic spun-bond non-woven fabric, hydrophilic hot-press non-woven fabric, hot-air non-woven fabric, spun-lace non-woven fabric, and porous plastic film.

10. The absorbent article as claimed in any one of claims 6 to 9, **characterized in that** said liquid-permeable backsheet layer (3) is made of one of a fresh material, a recycled material and a combination thereof, and includes at least one selected from the group consisting of hydrophilic woven fabric, hydrophilic spun-bond non-woven fabric, hydrophilic hot-press non-woven fabric, hot-air non-woven fabric, spun-lace non-woven fabric, and porous plastic film.

11. The absorbent article as claimed in any one of claims 1 to 10, **characterized in that** said liquid-absorbing body (41) is made of one of a fresh material, a recycled material and a combination thereof, and includes at least one selected from the group consisting of superabsorbent polymer sheet, liquid-absorbing polymer powder, paper pulp, cotton, and polyurethane foam.
